# EUROPEAN PATENT APPLICATION

(11) **EP 4 026 519 A1**
(43) Date of publication of application: **13.07.2022**
(21) Application number: 22150292.5
(22) Date of filing: 05.01.2022
(51) Int. Cl.: A61F 2/24

(54) **SEGMENTED ANNULAR REDUCTION SYSTEMS AND METHODS**

(30) Priority: 06.01.2021 US 202163134215 P; 15.11.2021 US 202117526440
(71) Applicant: Medtronic, Inc., Minneapolis, MN 55432 (US)
(72) Inventor: DORFF, Caitlin M., Minneapolis (US)
(74) Representative: Zimmermann & Partner Patentanwälte mbB

(57) **Abstract**

Aspects include a system that includes an annuloplasty device having a plurality of segments. Each segment includes first and second ends, a flexible body, a pawl at the second end, and a plurality of teeth positioned along the body. At least ones of the pawls and teeth are engaged in a manner such that the plurality of segments form a closed loop. The system can further include a pull wire releasably secured to at least one of the plurality of segments. Methods of reducing dimensions of an annulus, such as a mitral valve annulus, using the systems of the disclosure are also disclosed.

## Description

### CROSS-REFERENCE TO RELATED APPLICATION

This Non-Provisional Patent Application claims the benefit of the filing date of U.S. Provisional Patent Application Serial Number 63/134,215, filed January 6, 2021, the entire teachings of which are incorporated herein by reference.

### FIELD

The present technology is generally related to systems for and methods of reducing annular dimensions such as that of a mitral valve annulus or tricuspid valve annulus.

### BACKGROUND

Generally, the anatomy and physiology of the human heart is well known. Of the four one-way valves in the heart, the two inlet valves are the mitral valve of the left side of the heart, and the tricuspid valve on the right side of the heart. The tricuspid valve is located between the right atrium and the right ventricle. The three leaflets of the tricuspid valve laterally terminate at the tricuspid annulus. Blood flows from the superior and inferior vena cava into the right atrium, then through the tricuspid valve during diastole to fill the right ventricle. During ventricular systole, the tricuspid valve is closed and blood is ejected through the pulmonary valve into the pulmonary artery and hence through the lungs. At the end of ventricular systole the pulmonary valve closes. Leaving the lungs, the now oxygenated blood flows into the left atrium and hence through the mitral valve into the left ventricle during ventricular diastole. Finally, at ventricular systole the mitral valve closes and blood is ejected through the aortic valve into the aorta. However, should the mitral valve become regurgitant due to disease then some percentage of the left ventricular stroke volume will flow backwards through the mitral valve into the left atrium. This regurgitation causes the left atrial pressure to rise, in turn causing pulmonary artery pressure to rise, which is reflected back to the right ventricular pressure.

Typically, to treat a patient with functional mitral regurgitation, a physician places an annuloplasty ring on the mitral annulus to reduce the circumference and septal-lateral diameter of the annulus. In degenerative mitral regurgitation patients, annuloplasty rings are utilized to stabilize the mitral annulus, not reduce the annular circumference.

The present disclosure addresses problems and limitations associated with the related art.

### SUMMARY

The techniques of this disclosure generally relate to systems for and methods of reducing annular dimensions of an annulus, such as a mitral valve annulus. Such systems and methods can be used to treat functional mitral regurgitation, for example. Various embodiments of the disclosure enable selective plication of annulus tissue and also prevent potential migration of the annuloplasty ring between tissue plications and during post-procedural annular dilation.

In one aspect, the present disclosure provides a system that includes an annuloplasty device having a plurality of segments. Each segment includes first and second ends, a flexible body, a pawl at the second end, and a plurality of teeth positioned along the body. At least ones of the pawls and teeth are engaged in a manner such that the plurality of segments form a closed loop. The system can further include a pull wire releasably secured to at least one of the plurality of segments.

In another aspect, the disclosure provides a method including the steps of providing a mitral valve annulus of a patient and anchoring a plurality of segments of an annuloplasty device to the mitral valve annulus. The plurality of segments include a first segment and a second segment. Each segment has first and second ends, a flexible body, a pawl at the second end, and a plurality of teeth positioned along the body. Ones of the pawls and teeth are engageable such that the plurality of segments form a closed loop. A pull wire is releasably secured to the second end of the first segment. The method further includes proximally pulling the pull wire to draw the first end of the first segment through the pawl of the second segment.

Aspects and embodiments of the invention may be defined by the following clauses.

Clause 1. A system comprising:
an annuloplasty device including a plurality of segments; wherein each segment includes: first and second ends, a flexible body, a pawl at the second end, and a plurality of teeth positioned along the body; wherein at least ones of the pawls and teeth are engaged in a manner such that the plurality of segments form a closed loop; and
a pull wire releasably secured to at least one of the plurality of segments.

Clause 2. The system of clause 1, further comprising a delivery catheter in which the pull wire is housed.

Clause 3. The system of clause 2, wherein the delivery catheter includes a curved branch.

Clause 4. The system of clause 2 or 3, wherein the delivery catheter includes a branch in which the pull wire extends; wherein the branch has a distal end that abuts one pawl.

Clause 5. The system of any preceding clause, and in particular clause 1, wherein an anchor is secured to one pawl.

Clause 6. The system of any preceding clause, and in particular clause 5, wherein one anchor is secured to each pawl.

Clause 7. The system of any preceding clause, and in particular clause 1, wherein the pull wire extends through an opening in the pawl.

Clause 8. The system of any preceding clause, and in particular clause 1, wherein the pull wire is one of a plurality of pull wires.

Clause 9. The system of any preceding clause, and in particular clause 8, wherein one pull wire is releasably connected to every first end of each segment.

Clause 10. The system of any preceding clause, and in particular clause 1, wherein the pull wire includes a hook that is releasably engaged within an aperture in the first end of the second segment.

Clause 11. A method comprising:
providing a mitral valve annulus of a patient;
anchoring a plurality of segments of an annuloplasty device to the mitral valve annulus, the plurality of segments including a first segment and a second segment; wherein each segment includes: first and second ends, a flexible body, a pawl at the second end, and a plurality of teeth positioned along the body; wherein ones of the pawls and teeth are engageable such that the plurality of segments form a closed loop; wherein a pull wire is releasably secured to the first end of the first segment; and
proximally pulling the pull wire to draw the first end of the first segment through the pawl of the second segment.

Clause 12. The method of clause 11, wherein the pull wire is one of a plurality of pull wires, each pull wire being releasably secured to one first end of one of the plurality of segments; the method further comprising proximally pulling the pull wire to adjust a length between the pawl of the first segment and the pawl of the second segment.

Clause 13. The method of clause 12, wherein each pull wire is housed in a delivery catheter defining a plurality of branches; wherein each branch houses one single pull wire.

Clause 14. The method of clause 11, wherein the pull wire is housed in a delivery catheter defining a first branch and a second branch; the method including inserting the pull wire through the first branch and using the second branch as support for one pawl during the step of proximally pulling the pull wire.

Clause 15. The method of clause 11, wherein the plurality of segments form a closed loop.

Clause 16. The method of clause 11, wherein a diameter of the annuloplasty device is reduced by pulling each of a plurality of first ends of the plurality of segments through one respective pawl of the annuloplasty device.

Clause 17. The method of clause 11, wherein after the step of pulling the pull wire a portion of the first segment extends through the pawl of the second segment and over the second segment.

Clause 18. The method of clause 17, further comprising the step of severing the portion of the first segment and withdrawing it from the patient.

Clause 19. The method of clause 11, wherein each of the plurality of segments is anchored into the mitral valve annulus prior to interconnecting the plurality of segments to form a closed loop.

Clause 20. The method of clause 11, wherein each of the plurality of segments are interconnected to each other prior to the step of anchoring the plurality of segments of the annuloplasty device to the mitral valve annulus.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1A is a schematic top view of an annuloplasty device.
FIG. 1B is a partial, schematic side view of a system including the annuloplasty device of FIG. 1A anchored into tissue with a plurality of anchors and operatively connected to an adjustment catheter.
FIG. 1C is a partial, enlarged, schematic side view of the annuloplasty device of FIGS. 1A-1B having two segments in a pre-cinched arrangement.
FIG. 1D is a partial, enlarged, schematic side view of the two segments of FIG. 1C in a cinched arrangement.
FIG. 2 is a partial, perspective view of the annuloplasty device of FIGS. 1A-1D having an alternate anchor.
FIG. 3 is a partial, schematic side view of the adjustment catheter of FIG. 1B that can be used to cinch the segments of the annuloplasty device of FIGS. 1A-1D, for example.
FIG. 4 is a partial, schematic side view of an alternate adjustment catheter that can be used to cinch the segments of the annuloplasty device of FIGS. 1A-1D, for example.
FIGS. 5A-5B are partial, schematic side views of another adjustment catheter that can be used to cinch the segments of the annuloplasty device of FIGS. 1A-1D, for example.

### DETAILED DESCRIPTION

Specific embodiments of the present disclosure are now described with reference to the figures, wherein like reference numbers indicate identical or functionally similar elements. The terms "distal" and "proximal" are used in the following description with respect to a position or direction relative to the treating clinician. "Distal" or "distally" are a position distant from or in a direction away from the clinician. "Proximal" and "proximally" are a position near or in a direction toward the clinician.

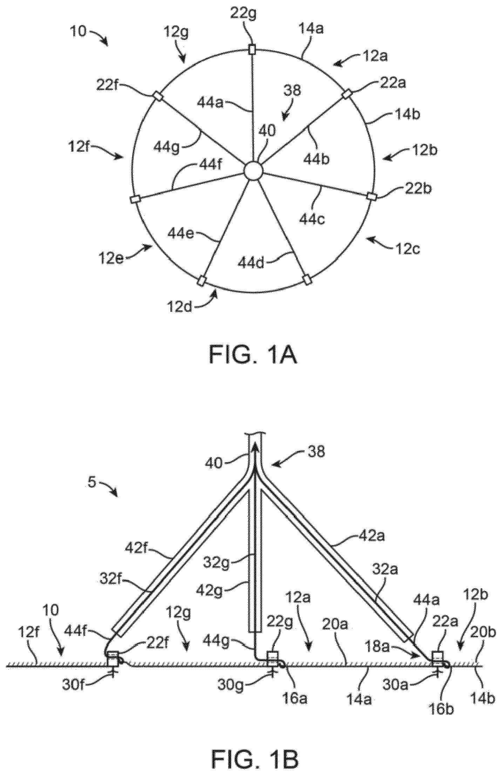

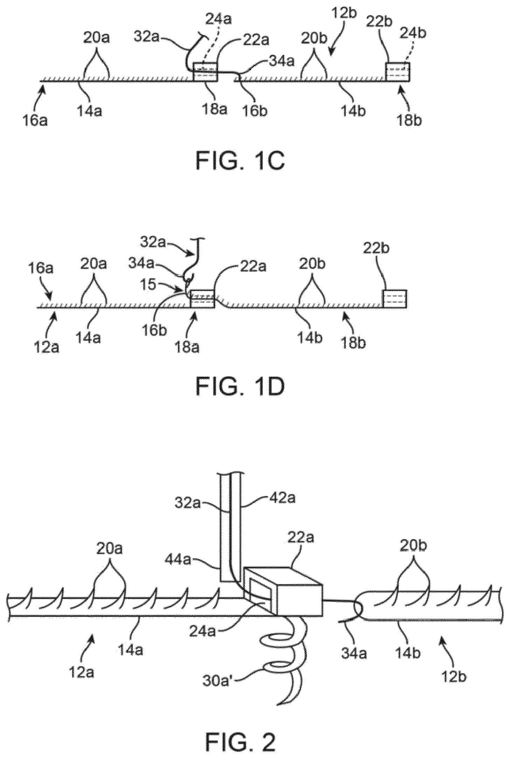

Figures 1A-1D collectively illustrate a system 5 including an annuloplasty device 10 formed of a plurality of interconnected segments 12a, 12b, 12c, 12d, 12e, 12f, 12g ("12a-12g"). The number of segments can vary, as desired. As shown, the annuloplasty device 10 has seven segments 12a-12g, however, fewer or greater segments are envisioned. Each segment 12a-12g includes a flexible body 14a, 14b having a first end 16a, 16b and a second end 18a, 18b. Only a first segment 12a and a second segment 12b are fully referenced in the figures for ease of illustration. Each segment 12a-12f can be identically configured or can vary, as desired. A plurality of teeth or engagement features 20a, 20b (generally referenced) are positioned along one side of the body 14a, 14b between the first end 16a, 16b and second end 18a, 18b. Each tooth 20a, 20b may include a protrusion or may be angled with respect to the body 14a, 14b. The first end 16a, 16b may be a free end and the second end 18a, 18b includes a pawl 22a, 22b. In one example, the pawls are integrally formed with the respective body (e.g., pawl 22a is integrally formed with the body 14a).

As is best shown in FIG. 2, each pawl 20a, 20b may have a longitudinal opening 24a, 24b extending from one side of the pawl 20a, 20b to the other. Each longitudinal opening 24a, 24b may be configured to receive the first end 16a, 16b of an adjacent segment in one direction but not the opposing direction. For example, the opening 24a of the pawl 22a of the first segment 12a may be sized and configured to receive the body 14b of the second segment 12b and upon insertion of the second segment body 14b into the opening 24a of the first segment 12a, the body 14b of the second segment 12b cannot be pulled back in the opposite direction. Generally, the plurality of teeth 20b of the second segment 12b and the pawl 22a of the first segment 12a may collectively form a ratchet. With this configuration, the plurality of segments 12a-12g can be interconnected, forming a ring or closed loop that can be reduced and maintained in dimension, which can prevent potential migration of the annuloplasty ring between tissue plications and during any potential post-procedural annular dilation. Once two adjacent segments are interconnected, they are essentially locked in the interconnected fashion. Each pawl and adjacent plurality of teeth can be configured similarly and function in a similar way. In one example, the plurality of teeth 20a, 20b extend along an entire length of the respective body 14a, 14b at specified intervals, to allow for more intermediate control and locking of annular ring size/cinch amount between two adjacent pawls 22a, 22b.

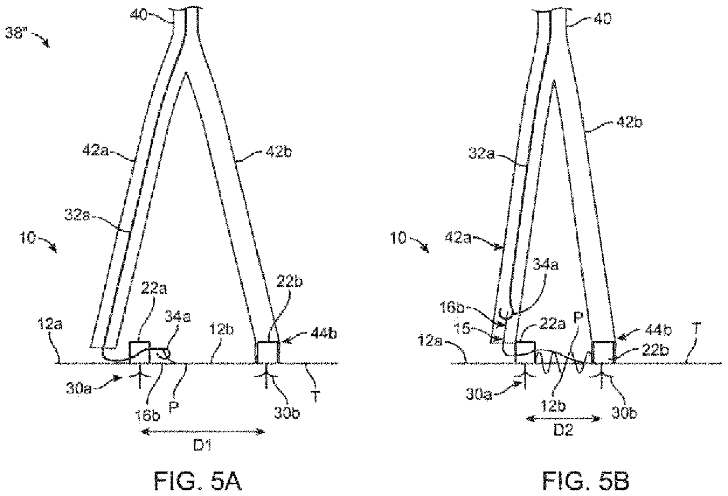

It is envisioned that the annuloplasty device 10 can be anchored or otherwise secured to a valve annulus (see also, FIGS. 5A-5B and related disclosure), such as a mitral valve annulus in a variety of manners. In one example, the annuloplasty device 10 is secured to the valve annulus prior to cinching of the annuloplasty device 10. In various embodiments, the annuloplasty device is secured with one or more anchors that can be provided as part of system 5 or annuloplasty device 10. The anchors can have a variety of configurations including barbs, prongs, shape memory elements or helical members. In the example of FIG. 1B, one anchor 30a, 30b is positioned at each pawl 22a, 22b. In various embodiments, each anchor 30a, 30b is secured to one pawl 22a, 22b. Figure 1B illustrates anchors 30a, 30b each having a plurality of shape memory prongs that can be inserted into tissue in an elongated (i.e. generally linear arrangement) and then the prongs will spring into the illustrated natural arrangement once released into the tissue. In other embodiments, such as that of FIG. 2, one or more anchors 30a' may be helical for rotational advancement into tissue. In instances where the anchor is rotatably advanced into tissue, each segment (e.g., segments 12a-12g) may be anchored or attached to the valve annulus or other tissue prior to being formed into an interconnected ring or loop. In other words, segments 12a-12g can be anchored to tissue individually and disconnected from each other. In various methods, once the annuloplasty device 10 is secured to the valve annulus, one or more segments 12a-12g are independently cinched to a desired degree and are automatically locked or maintained in the cinched arrangement upon cinching due to the one-way movement restriction of the ratchet configuration formed by respective pawls and teeth.

Cinching of each segment 12a-12g can be conducted, for example, by actively pulling a respective pull wire (e.g., pull wires 32a, 32b) releasably secured to one respective segment (e.g., segments 12a, 12b) and provided as part of system 5. In one example, the pull wire 32a, 32b includes a hook 34a, 34b that can be configured to releasably engage the first end of the body of an adjacent segment. In operation, each pull wire may be threaded through the longitudinal opening in the pawl adjacent the respective second end so that proximal pulling of the pull wire will correspondingly pull the body through the pawl. For example, as shown in FIGS. 1C-1D, pull wire 32a is threaded through the opening 24a in the pawl 22a of the first segment 12a. The pull wire 32a may be releasably engaged with the second end 18a of the second segment 12b. Upon proximal retraction of the pull wire 32a, the second segment 12b is drawn through the opening 24a to shorten the distance between the pawl 22a of the first segment 22a and the pawl 22b of the second segment 12b, effectively "cinching" the second segment 12b and tissue anchored proximate thereto (see also FIGS. 5A-5B and related disclosure).

The pull wires 32a, 32b can be protected and housed within an adjustment catheter 38 (best shown in FIG. 1B), which can be provided as part of system 5. In one example, the adjustment catheter 38 can include one stem catheter 40 that distally terminates at a branch 42a, 42g, 42f for each pull wire 32a, 32g, 32f, which directs one respective pull wire 32a, 32g, 32f to one respective pawl (see pull wires 32a, 32f, 32g and pawls 22a, 22f, 22g in FIG. 1B; only three branches, pull wires and segments are shown for ease of illustration, however, the remaining branches, pull wires and segments can be similarly configured and operated in an identical manner). Each branch 42a, 42f, 42g terminates at a distal end 44a, 44f, 44g. In the example of FIG. 1B, each distal end 44a, 44f, 44g can be positioned proximally and over one pawl 22a, 22f, 22g. In this example, a pivot-point of each pull wire 32a, 32f, 32g is above (in the perspective shown) each anchor 30a, 30f, 30g and pawl 22a, 22f, 22g.

In one example method, the annuloplasty device is provided and anchored to a mitral valve annulus so that the annuloplasty device circumscribes the annulus and cannot move with respect to the annulus. An adjustment catheter of any of the types disclosed herein houses at least one pull wire, each of which is releasably secured to a second end of a second segment and passing through the pawl of a first segment. One or more pull wires are pulled proximally to pull the respective second end of the segment into the pawl until the desired amount of shortening between the pawl and adjacent pawl at the first end of the segment is achieved. Once the desired shortening of the respective segment is achieved, the pull wire is disengaged from the respective first end. Turning again to the example of FIGS. 1C-1D, once the desired amount of cinching of the second segment 12b has been conducted, the pull wire 32b can be selectively detached from the body 14b by unhooking the hook 34a from the first end 16b. Any other releasable attachment methods that provide similar function are considered within the scope of the present disclosure. It is envisioned that the excess portion 15 of body 14b pulled through the pawl 22a can remain attached. In some embodiments, the excess portion 15 of the body 14b can be severed and removed. The pull wire can be directed to another pawl with the adjustment assembly and the process can be repeated until the desired adjustment of the annuloplasty ring is achieved. In one embodiment, multiple pull wires are provided and used to adjust multiple segments separately, either simultaneously or in sequence.

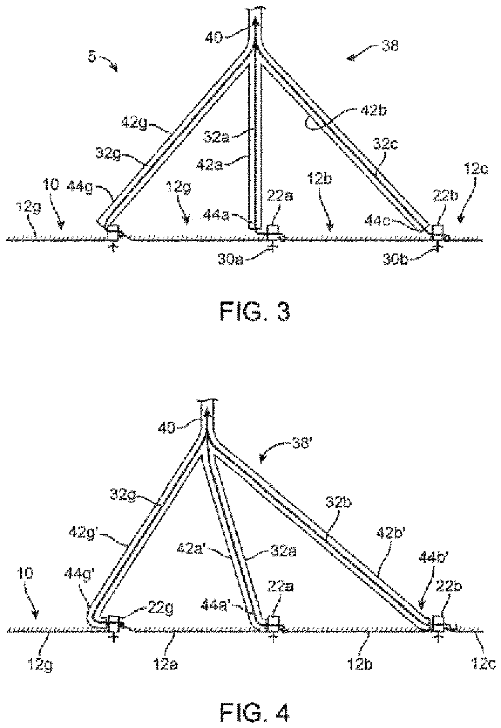

In another example shown in FIG. 3, during cinching, the distal end 44a, 44b, 44g of each branch 42a, 42b, 42g can be positioned adjacent the opening 24a, 24b of the pawl 22a, 22b, 22g so that a pull force vector of each pull wire 32a, 32b, 32g is parallel to the valve annulus and will not apply a dehiscence force to the anchor 30a, 30b, 30g. As at least partially indicated with like reference numerals, the embodiment of FIG. 3 can be identically configured, function and be implanted in the same manner as other embodiments disclosed herein except as explicitly stated.

In yet another example shown in FIG. 4, a distal end 44a', 44b', 44g' of each branch 42a', 42b', 42g' can be curved so that the branch 42a', 42b', 42g' is non-linear along its length. The distal end 44a', 44b', 44g' is curved to lead the respective pull wire 32a, 32b, 32g into the respective pawl opening (e.g., 24a, 24b; see also FIG. 2) so that the direction of the pull-force vector would avoid anchor dehiscence. In this embodiment, the distal ends 44a', 44b', 44g' of each branch 42a', 42b', 42g' can also be configured to serve as a backstop for each respective anchor 30a, 30b, 30g to provide a reactional force to allow the second end of the body to move though the pawl opening without the anchor 30a, 30b, 30g moving with the body. For example, the distal ends of 44a' of the branch 42a' may serve as a backstop for the anchor 30a to provide a reactional force to allow the first end 16b of the body 14b of the second segment 12b to move though the pawl opening 24a without the anchor 30a moving with the body 14b. As at least partially indicated with like reference numerals, the embodiment of FIG. 4 can be otherwise identically configured, function and be implanted in the same manner as other embodiments disclosed herein except as explicitly stated.

Referring in addition to FIGS. 5A-5B, which illustrate an alternate adjustment catheter 38" and methods utilizing only two branches 42a, 42b extending from the stem 40. Each branch 42a, 42b can optionally take the configuration of branches 42a', 42b' disclosed above. In one example, a single pull wire 32a could be inserted through one branch 42a while adjacent branch 42b can receive pawl 22b to provide support as the pull wire 32a is proximally pulled. In one example, a single pull wire 32a could be moved sequentially from one branch 42a to the other branch 42b to adjust the respective segment(s) (e.g., segments 12a-12f) in a manner such as that disclosed above. The branches 42a, 42b can then be repositioned at additional pawls to interconnect the pull wire 32a to additional segments for cinching. In another example, segment 12b is cinched to correspondingly cinch portion P of tissue T secured thereto. Such cinching is accomplished by reducing a distance D1 between adjacent pawls 22a, 22b to a smaller/reduced distance D2). The excess portion 15 can be either severed outside of the respective branch 42a or can be drawn into the respective branch 42a be severed and removed from the patient through the adjustment catheter 38". Or, the excess portion 15 can be left in place. The adjustment catheter 38" could then be repositioned at a second set of pawls, and then a third set of pawls, and so on, as desired, to selectively cinch desired segments. In some embodiments the distal end 44b of the branch 42b that is opposite the pull wire can receive the adjacent pawl 22b to provide support during cinching. As at least partially indicated with like reference numerals, the embodiment of FIGS. 5A-5B can be identically configured, function and be adjusted in the same manner as other embodiments disclosed herein except as explicitly stated.

In yet another example of the disclosure, the adjustment catheter 38" can include a single branch 42a that can be a continuous extension of stem 40 or angled with respect to stem 40. In this example, since branch 42b would not be provided to lend additional stabilizing support to the system during cinching, stabilization and support can be achieved by abutting the distal end 44a of the branch 42a against a proximal end of the respective pawl (as previously described, to provide a reactionary force), either in embodiments having curved distal end (e.g., FIG. 4) or non-curved distal end (e.g., FIG. 3). In various examples of the disclosure, one or more flexible segments (e.g., flexible segment 12b) is already partially threaded through one pawl (e.g., pawl 22a), so the pull wire 32a only needs to hook onto the end of the flexible segment 12b for cinching of each flexible segment, without having to also weave all the way through one respective pawl to reach the respective flexible segment. Similarly, in the multi-branch embodiments shown in FIGS. 1A-4, the pull wires can optionally be woven through respective pawls outside of the patient pre-implantation, but with a single-arm or dual-arm "tool-style" catheter that moves around and cinches each flexible segment in a serial fashion. It is envisioned that pre-threading of the flexible segments at least partially through the pawls may ease navigation and technical feasibility of implantation of such embodiments.

Aspects of the disclosure provide numerous advantages over known devices and techniques. Particularly, aspects of the disclosure allow for variable localized cinch amounts of the device around the annulus that provide a more patient-specific and tailored treatment corresponding with varying amounts of annulus dilatation. Further, aspects of the disclosure provide systems to simultaneously cinch and locking, rather than locking needing to be accomplished in a separate step. Simultaneous cinching and locking reduces procedural complexity and time.

It should be understood that various aspects disclosed herein may be combined in different combinations than the combinations specifically presented in the description and accompanying drawings. Each embodiment and each aspect so defined may be combined with any other embodiment or with any other aspect unless clearly indicated to the contrary. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

## Claims

1. A system comprising:
an annuloplasty device including a plurality of segments; wherein each segment includes: first and second ends, a flexible body, a pawl at the second end, and a plurality of teeth positioned along the body; wherein at least ones of the pawls and teeth are engaged in a manner such that the plurality of segments form a closed loop; and
a pull wire releasably secured to at least one of the plurality of segments.

2. The system of claim 1, further comprising a delivery catheter in which the pull wire is housed.

3. The system of claim 2, wherein the delivery catheter includes a curved branch.

4. The system of claim 2 or 3, wherein the delivery catheter includes a branch in which the pull wire extends; in particular wherein the branch has a distal end that abuts one pawl.

5. The system of any preceding claim, wherein an anchor is secured to one pawl.

6. The system of any preceding claim, wherein one anchor is secured to each pawl.

7. The system of any preceding claim, wherein the pull wire extends through an opening in the pawl.

8. The system of any preceding claim, wherein the pull wire is one of a plurality of pull wires.

9. The system of any preceding claim, wherein one pull wire is releasably connected to every first end of each segment.

10. The system of any preceding claim, wherein the pull wire includes a hook that is releasably engaged within an aperture in the first end of the second segment.

11. A method comprising:
providing a mitral valve annulus of a patient;
anchoring a plurality of segments of an annuloplasty device to the mitral valve annulus, the plurality of segments including a first segment and a second segment; wherein each segment includes: first and second ends, a flexible body, a pawl at the second end, and a plurality of teeth positioned along the body; wherein ones of the pawls and teeth are engageable such that the plurality of segments form a closed loop; wherein a pull wire is releasably secured to the first end of the first segment; and
proximally pulling the pull wire to draw the first end of the first segment through the pawl of the second segment.

12. The method of claim 11, wherein the pull wire is one of a plurality of pull wires, each pull wire being releasably secured to one first end of one of the plurality of segments; the method further comprising proximally pulling the pull wire to adjust a length between the pawl of the first segment and the pawl of the second segment.

13. The method of claim 12, wherein each pull wire is housed in a delivery catheter defining a plurality of branches; wherein each branch houses one single pull wire.

14. The method of claim 11, wherein the pull wire is housed in a delivery catheter defining a first branch and a second branch; the method including inserting the pull wire through the first branch and using the second branch as support for one pawl during the step of proximally pulling the pull wire.

15. The method of claim 11, wherein the plurality of segments form a closed loop.
